# EUROPEAN PATENT APPLICATION

(11) **EP 3 406 700 A1**
(43) Date of publication of application: **28.11.2018**
(21) Application number: 17741466.1
(22) Date of filing: 19.01.2017
(51) Int. Cl.: C12M 1/00, B29C 33/38, B29C 59/02

(54) **CELL CULTURE SUBSTRATE AND METHOD FOR PRODUCING SAME**

(30) Priority: 20.01.2016 JP 2016008809
(71) Applicant: Soken Chemical & Engineering Co., Ltd., Toshima-ku Tokyo 171-8531 (JP)
(72) Inventor: SUDA, Kaoru, Sayama-shi Saitama 350-1320 (JP); NIMIYA, Keisuke, Sayama-shi Saitama 350-1320 (JP); MIZAWA, Takahide, Sayama-shi Saitama 350-1320 (JP)
(74) Representative: Gulde & Partner
(86) International application number: PCT/JP2017/001689
(87) International publication number: WO 2017/126589

(57) **Abstract**

A composite pattern culture substrate which can be conveniently produced using readily available materials and can cultivate uniform spheroids with high viability.

According to some embodiments of the present invention, a cell culture substrate having an adhesive portion and a non-adhesive portion is provided, wherein the adhesive portion and the non-adhesive portion have a concave-convex shape, and the contact angle of pure water on the non-adhesive portion is larger than that of the adhesive portion.

## Description

### Technical Field

The present invention relates cell culture substrate and manufacturing method thereof.

### Background Art

Generally, in the field of drug development, a screening test is known in which a drug is administered to a cell mass (spheroid) cultured in a three-dimensional shape to measure its metabolic ability to the drug. In order to increase the efficiency of this metabolic test, in recent years it has been required to cultivate uniform spheroids with high viability using a culture container that can be conveniently prepared. As a container for spheroid culture, as disclosed in PTL 1, a substrate or the like having an adhesion region and an inhibition region for cells is used as a culture substrate.

### Citation List

### Patent Literature

[PLT1] JP2007-269973A

### Summary of Invention

### Technical Problem

Resins being able to cultivate relatively uniform spheroids and having a property that can be used for an adhesive region or an inhibition region are special and limited(PLT1). It is difficult to synthesize such a special resin, and it takes a long time and high cost.

Several embodiments of the present invention have been made in view of such circumstances and provide a cell culture substrate which can be conveniently produced using readily available materials and can cultivate uniform spheroids with high viability.

### Solution to Problem

According to some embodiments of the present invention, a cell culture substrate having an adhesive portion and a non-adhesive portion is provided, wherein the adhesive portion and the non-adhesive portion have a concave-convex shape, and the contact angle of pure water on the non-adhesive portion is larger than that of the adhesive portion.

The present inventors found out that it is possible to culture uniform spheroids with high viability by using a cell culture substrate comprising an adhesive portion having a concave-convex shape and highly hydrophobic non-adhesive portion having a concave-convex shape different from the adhesive portion even with readily available materials, and thus has come to complete the present invention.

Various embodiments of the present invention are exemplified below. Embodiments shown below may be combined with each other.

It is preferred that, pitch P₁ of the concave-convex shape of the adhesive portion is larger than pitch P₂ of the concave-convex shape of non-adhesive portion.

It is preferred that, the ratio P₁/P₂ of the pitch P₁ and the pitch P₂ is 2 or more.

It is preferred that, at least a part of the adhesive portion is separated by a partition portion, and the non-adhesive portion is formed on the partition portion.

It is preferred that, the non-adhesive portion has a plurality of columnar convex portion, the pitch at which the columnar convex portion of the non-adhesive portion is formed is 50 to 2000 nm, the columnar convex portion of the non-adhesive portion has an upper surface area of 1600 nm² to 4 µm², and the gap G₂ between two adjacent columnar convex portions is in the range of 10 to 1960 nm.

It is preferred that, the adhesive portion and the non-adhesive portion are made of a same material.

According to another embodiment of the present invention, a manufacturing method of the cell culture substrate is provided that includes a step of irradiating a photocurable resin composition coated on a substrate with an active energy ray to cure the resin composition to form concave-convex shapes of the adhesive portion and the non-adhesive portion.

It is preferred that, the concave-convex shapes of the adhesive portion and the non-adhesive portion are formed by a nanoimprint method using a mold.

### Brief Description of Drawings

Figs. 1A to 1C show a cell culture substrate 1 according to one embodiment of the present invention, wherein Fig. 1A is a plan view, Fig. 1B is a cross-sectional view taken along the line A-A, and Fig. 1C is a cross-sectional view showing a pattern in which the concave-convex shape according to another embodiment is inverted from the case of Fig. 1B.
Figs. 2A to 2B are enlarged views of a region X in Fig. 1B showing an adhesive portion 3 according to one embodiment of the present invention, wherein Fig. 2A is a plan view, and Fig. 2B is a cross-sectional view taken along the line B-B.
Figs. 3A to 3B are enlarged views of a region Y in Fig. 1B showing a non-adhesive portion 5 according to one embodiment of the present invention, wherein Fig. 3A is a plan view, Fig. 3B is a cross-sectional view taken along the line C-C.
Figs. 4A to 4B are cross-sectional views of the cell culture substrate 1 according to one embodiment of the present invention, wherein Fig. 4A shows an example having a partition portion 19, and Fig. 4B shows an example without a partition portion.
Figs. 5A to 5E show a transparent base 23 provided with light shielding part 21, wherein Fig. 5A is a plan view, Fig. 5B is a cross-sectional view taken along the line D-D, and Figs. 5C to 5E show another example of the method of forming a light shielding pattern 3.
Figs. 6A to 6C are cross-sectional views showing a first transferred resin layer forming step of the present invention.
Figs. 7A to 7D are cross-sectional views showing an adhesive portion resin layer forming step of the present invention.
Figs. 8A to 8E are cross-sectional views showing a composite resin layer forming step of the present inventio.
Fig. 9 shows SEM images A to D of samples 1 to 4.

### Description of Embodiments

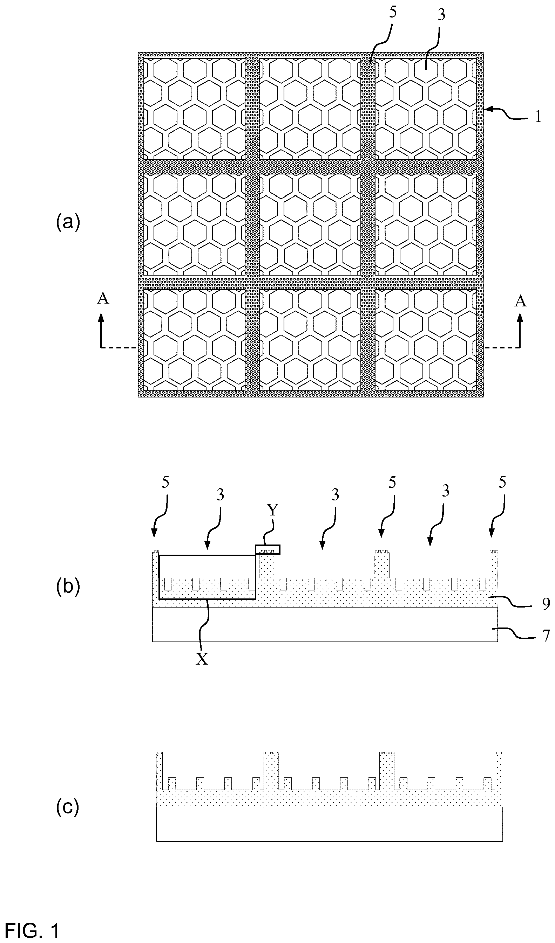

Preferred embodiments of the present invention are specifically described below with reference to Figs. 1 to 4.

### 1. Cell Culture Substrate

As illustrated in Fig. 1B, a cell culture substrate 1 according to one embodiment of the present invention comprises a base 7 and a resin layer 9 on at least one surface of the base 7, and the resin layer 9 has an adhesive portion 3 and a non-adhesive portion 5. The concave-convex shape of the adhesive portion 3 and the non-adhesive portion 5 shown in Fig. 1B may have a shape in which the concave and convex of only one of the adhesive portion 3 and non-adhesive portion 5 is reversed, or may have a shape as shown in Fig. 1C in which the concave and convex of both are reversed.

It is preferred that, the non-adhesive portion 5 is arranged in such a manner that it surrounds the adhesive portion 3, and the resin layer 9 is divided into the region of adhesive portion 3 and the region of non-adhesive portion 5. However, it is not necessarily surrounded without a break, and a part may be discontinued.

The shape of the region of the adhesive portion 3 is not particularly limited, and may be a circle, a polygon, etc. For example, an embodiment in which a square region as shown in Fig. 1A is formed is conceivable.

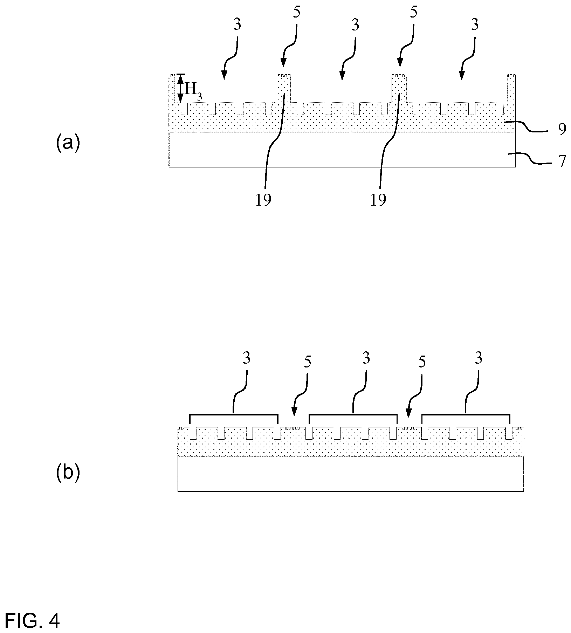

Further, as illustrated in Fig. 4A, the cell culture substrate 1 may have a partition portion 19 that separates at least a part of the adhesive portion. Further, as illustrated in 4B, the cell culture substrate 1 may not have the partition portion 19. In order to prevent detachment of the cells from the base during replacement of the medium or the like, it is preferable to have the partition portion 19.

### < Base 7>

The material of the base 7 is preferably, but not particularly limited to a transparent base such as a resin base and a quartz base. From the viewpoint of flexibility, a resin base is more preferable. A resin constituting the resin base is made of, for example, one selected from the group consisting of polyethylene terephthalate, polycarbonate, polyester, polyolefin, polyimide, polysulfone, polyether sulfone, cyclic polyolefin, and polyethylene naphthalate. Further, the base 7 is preferably in the form of a film having flexibility, and preferably has a thickness ranging from 25 to 500 µm.

### < Resin Layer 9>

A resin constituting the resin layer 9 is not particularly limited, but it is preferable that the resin layer 9 is made of an inexpensive resin that is relatively easy to obtain and synthesize, such as acrylic resin, methacrylic resin, styrene resin, olefin resin, polycarbonate resin, polyester resin, epoxy resin, and silicone resin. The types of the resin materials of the adhesive portion 3 and the non-adhesive portion 5 may be different, but from the viewpoint of easiness of manufacture and efficiency, it is preferable to be the same.

### < Adhesive Portion 3>

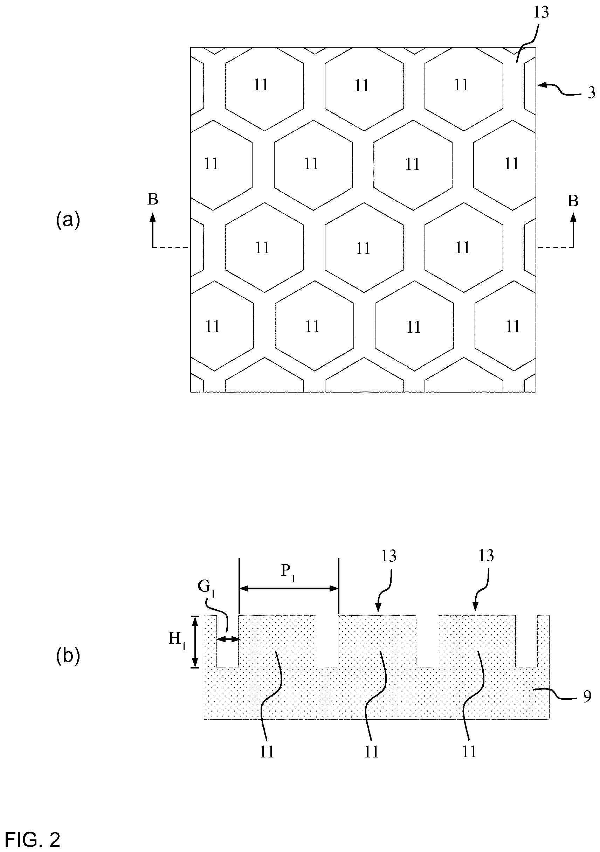

As illustrated in Fig. 2, adhesive portion 3 has a concave-convex shape formed by a plurality of adhesive convex portions 11 and adhesive concave portions 13. The convex portion and the concave portion may be inverted shapes.

### <Adhesive Convex Portion 11 and Adhesive Concave Portion 13>

The shape of the adhesive convex portion 11 is not particularly limited, but examples thereof include a column shape (a circular column shape, a polygonal columnar shape, etc.), a truncated cone, a microlens, and the like. The shape of the adhesive concave portion 13 is also not particularly limited, but examples thereof include a hole hollowed out in a shape such as a column shape (a circular column shape, a polygonal columnar shape, etc.), a truncated cone, a microlens, and the like.

The pitch P₁ at which the adhesive convex portion 11 or the adhesive concave portion 13 is formed may be within a range having adhesiveness to a target cell, and is, for example, 2 to 50 µm, preferably 5 to 20 µm, and more preferably 7 to 15 µm. If the pitch P1 is too small, cells are difficult to adhere. Also, if the pitch P1 is too large, cells are difficult to form spheroids. Specifically, the pitch P₁ is, for example, 2, 3, 4, 5, 6, 7, 8, 9, 10, 11, 12, 13, 14, 15, 16, 17, 18, 19, 20, 25, 30, 35, 40, 45, 50 µm, and may be within the range between any two of the numerical values exemplified here. The concave-convex shape formed by the adhesive convex portion 11 and the adhesive concave portion 13 may be regular or irregular, but is preferably regular from the viewpoint of working efficiency. In the case where the concave-convex shape is formed regularly, the distance between the tips of many convex portions or concave portions constituting the concave-convex shape is defined as "pitch". In the case where the concave-convex shape is formed irregularly, the average value of the distances between the tips of the many convex portions or concave portions constituting the concave-convex shape is defined as "pitch".

The height H₁ of the adhesive convex portion 11 is not particularly limited, but is, for example, 1 to 20 µm, preferably 1 to 10 µm, more preferably 1 to 5 µm. If the height H₁ is too low, cells are less likely to form spheroids, and if it is too high, the adhesive convex portion 11 tends to collapse easily.

A typical example of the adhesive portion has a plurality of columnar convex portions. The area of the upper surface of the columnar convex portion of the adhesive portion may be within a range having adhesiveness to the target cell, and is, for example, 1 to 2400 µm², preferably 16 to 360 µm², more preferably 36 to 196 µm². The gap G₁ between two adjacent columnar convex portions may be within a range having adhesiveness to a target cell, and is, for example, 1 to 49 µm, preferably 4 to 19 µm, more preferably 6 to 14 µm.

### <Non-adhesive Portion 5>

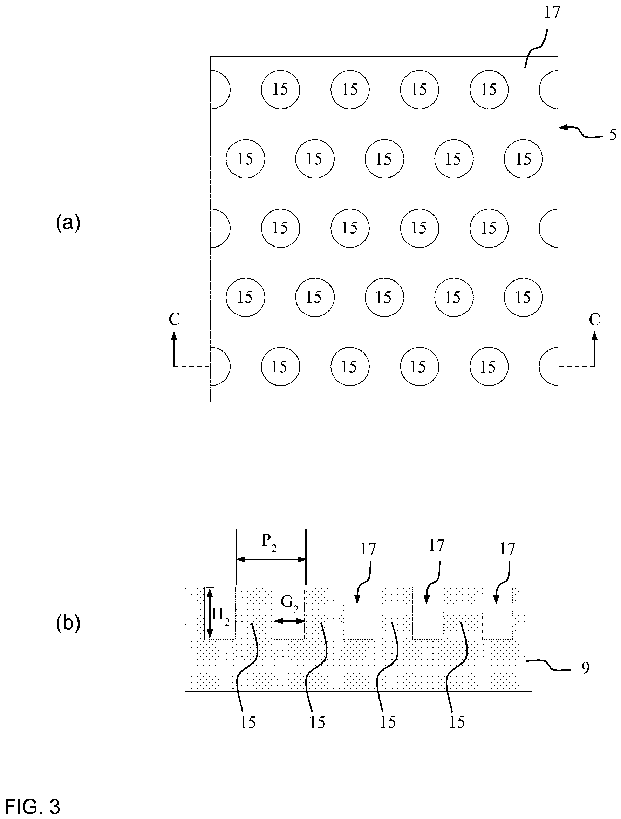

As illustrated in Fig. 3, the adhesive portion 3 has a concave-convex shape formed by a plurality of non-adhesive convex portion 15 and non-adhesive concave portion 17. The convex portion and the concave portion may be inverted.

Further, the contact angle of water on the non-adhesive portion 5 is not particularly limited but is larger than that of the adhesive portion 3. If the contact angle is larger than that of the adhesive portion 3, cells are difficult to adhere to the non-adhesive portion 5 and are easy to form spheroids. The contact angle of water on the non-adhesive portion 5 is, for example, 90 to 180 °, preferably 105 to 180 °, more preferably 110 to 180 °, further preferably 115 to 180 °.

### <Non-adhesive Convex Portion 15 and Non-adhesive Concave Portion 17>

The shape of the non-adhesive convex portion 15 is not particularly limited, but examples thereof include a column shape (a circular column shape, a polygonal columnar shape, etc.), a truncated cone, a microlens, and the like. The shape of the non-adhesive concave portion 17 is also not particularly limited, but examples thereof include a hole hollowed out in a shape such as a column shape (a circular column shape, a polygonal columnar shape, etc.), a truncated cone, a microlens, and the like.

The pitch P₂ at which the non-adhesive convex portion 15 or the non-adhesive concave portion 17 is formed is 50 to 2000 nm, preferably 100 to 1000 nm, more preferably 150 to 800 nm. If the pitch P₂ is too small, cells can not recognize the concave and convex, and there is no discrimination between adhesiveness and non-adhesiveness. If P₂ is too large, cells tend to adhere. Specifically, the pitch P₂ is, for example, 50, 60, 70, 80, 90, 100, 150, 200, 250, 300, 350, 400, 450, 500, 550, 600, 650, 700, 750, 800, 850, 900, 950, 1000, 1250, 1500, 1750, 2000 nm, and may be within the range between any two of the numerical values exemplified here. The concave-convex shape formed by the non-adhesive convex portion 15 and the non-adhesive concave portion 17 may be regular or irregular, but is preferably regular from the viewpoint of working efficiency. In the case where the concave-convex shape is formed regularly, the distance between the tips of many convex portions or concave portions constituting the concave-convex shape is defined as "pitch". In the case where the concave-convex shape is formed irregularly, the average value of the distances between the tips of the many convex portions or concave portions constituting the concave-convex shape is defined as "pitch".

The pitch P₁ of the concave-convex shape of the adhesive portion 3 is larger than the pitch P₂ of the concave-convex shape of the non-adhesive portion 5. The larger the pitch, the smaller the contact angle of water and the higher the adhesiveness to cells tends to be. On the other hand, the smaller the pitch, the larger the contact angle of water and the lower the adhesiveness to cells tend to be. Therefore, by surrounding the region of the concave-convex shape with a large pitch by the region of the concave-convex shape with a small pitch, spheroid can be easily formed. Preferably, the ratio P₁/P₂ of the pitch P₁ and the pitch P₂ is 2 or more, more preferably 5 or more, further preferably 10 or more. When P₁/P₂ is large, there is a tendency that the difference in adhesiveness tends to be large, and it is possible to cultivate spheroids more efficiently because the cells are more likely to gather. The upper limit of P₁/P₂ is not particularly specified, but is, for example, 200.

The height H₂ of the non-adhesive convex portion 15 is not particularly limited, but is, for example, 50 to 2000 nm. The height H₂ is preferably 100 to 1000 nm, and more preferably 150 to 800 nm. If the height H₂ is too low, cells tend to adhere, and if it is too high, the non-adhesive convex portion 15 tends to collapse easily.

A typical example of the the non-adhesive portion has a plurality of columnar convex portions, more specifically, a plurality of convex portions with circular column shape. The area of the upper surface of the columnar convex portion of the non-adhesive portion may be within a range in which the adhesion to the target cell is low, and is, for example, 1600 nm² to 4 µm². This area is preferably 810 nm² to 1 µm², more preferably 0.02 to 0.6 µm². The gap G₂ between two adjacent columnar convex portions may be within a range in which the adhesion to the target cell is low, and is, for example, 10 to 1960 nm, preferably 60 to 960 nm, and more preferably 110 to 760 nm.

A fluorine atom-containing layer may be provided in such a manner that the fluorine atom-containing layer covers the non-adhesive convex portion 15 and non-adhesive concave portion 17. fluorine atom-containing layer may contain a fluorine atom, and its thickness and composition are not limited. By providing the fluorine atom-containing layer it becomes difficult for the cells to adhere. The fluorine atom-containing layer preferably contains a fluorine-containing group. The fluorine-containing group is, in one example, a perfluoroalkyl group, more specifically a perfluoroalkylsilane group. The number of carbon atoms of the perfluoroalkyl group is, for example, 1 to 10, specifically 1, 2, 3, 4, 5, 6, 7, 8, 9, 10, and it may be within the range between any two of the numerical values exemplified here. fluorine-containing group is preferably chemically bonded to the non-adhesive convex portion 15 and the non-adhesive concave portion 17, or the inorganic film. In general, the inorganic film has high adhesiveness to the resin layer 9, and the fluorine-containing group tends to form a strong chemical bond to the inorganic film. Therefore, by providing the inorganic film between the non-adhesive convex portion 15, the non-adhesive concave portion 17 and the fluorine atom-containing layer, the fluorine atom-containing layer is firmly held on the non-adhesive convex portion 15 and the non-adhesive concave portion 17. Examples of the inorganic film include an inorganic oxide film, an inorganic nitride film, an inorganic oxynitride film, and the like. Examples of the inorganic element constituting the inorganic film include silicon and aluminum. The inorganic film is, for example, a silicon dioxide film or an aluminum oxide film. The thickness of the inorganic film is not particularly limited but is, for example, 1 to 20 nm.

The fluorine atom-containing layer, in one example, can be formed by forming an inorganic film on the non-adhesive convex portion 15 and the non-adhesive concave portion 17, and reacting the inorganic film with a fluorine-containing silane coupling agent. The fluorine-containing silane coupling agent is, for example, a perfluoroalkyltrialkoxy (methoxy, ethoxy, etc.) silane. Since even when a fluorine-containing silane coupling agent is allowed to act on the non-adhesive convex portion 15 and the non-adhesive concave portion 17 without forming an inorganic film, it is preferable to previously form the inorganic film on the non-adhesive convex portion 15 and the non-adhesive concave portion 17. An example of the fluorine-containing silane coupling agent is OPTOOL DSX (manufactured by Daikin Industries, Ltd.).

### < Region of Adhesive Portion 3 >

The area of the region of the adhesive portion 3 may be appropriately adjusted according to the type of cells to be cultured and the intended use of the spheroid, and the area is not particularly limited, but is, for example, 25 to 1000000 µm², preferably 100 to 250000 µm², more preferably 2500 to 40000 µm². When the shape of the region of the adhesive portion 3 is a square, the length of one side is 5 to 1000 µm, preferably 10 to 500 µm, more preferably 50 to 200 µm.

### < Region of Non-adhesive Portion 5 >

If the region of the non-adhesive portion 5 is too large with respect to the region of the adhesive portion 3, spheroids are difficult to form because the cells are difficult to move to the region of the adhesive portion 3. Therefore, the ratio of the area of the region of the non-adhesive portion 5 to the total area of region of the adhesive portion 3 and the region of the non-adhesive portion 5 is preferably, for example, 80% or less, more preferably 50% or less, further preferably 25% or less.

### <Partition portion 19>

As illustrated in Fig. 4A, in the case of having the partition portion 19 that separates at least a part of the adhesive portion 3, preferably, the partition portion 19 is arranged in such a manner that it surrounds the adhesive portion 3 to form a region of the adhesive portion 3. However, it is not necessarily surrounded without a break, and a part may be discontinued. The shape of the region of the adhesive portion 3 is not particularly limited but may be a circle or a polygon such as a quadrangle. Preferably, the non-adhesive portion 5 is formed on the partition portion. The non-adhesive portion 5 may be formed on a part of the partition portion, and is preferably formed in such a manner that the non-adhesive portion 5 covers at least most of the upper surface of the partition portion 19. The non-adhesive portion 5 may cover the entire surface of the partition portion 19.

The height H₃ of the partition portion 19 is not particularly limited, but is, for example, 5 to 100 µm, preferably 10 to 50 µm, more preferably 15 to 25 µm. If the height H₃ is too low, the cells tend to be peeled off due to water flow or the like. If the height H₃ is too high, oxygen supply can not be sufficiently carried out and the cell viability is lowered. As illustrated in Fig. 4A, the height H₃ is the height from the upper end of the adhesive convex portion 11 to the upper end of the partition portion including the non-adhesive portion.

### 2. Manufacturing Method of Cell Culture Substrate

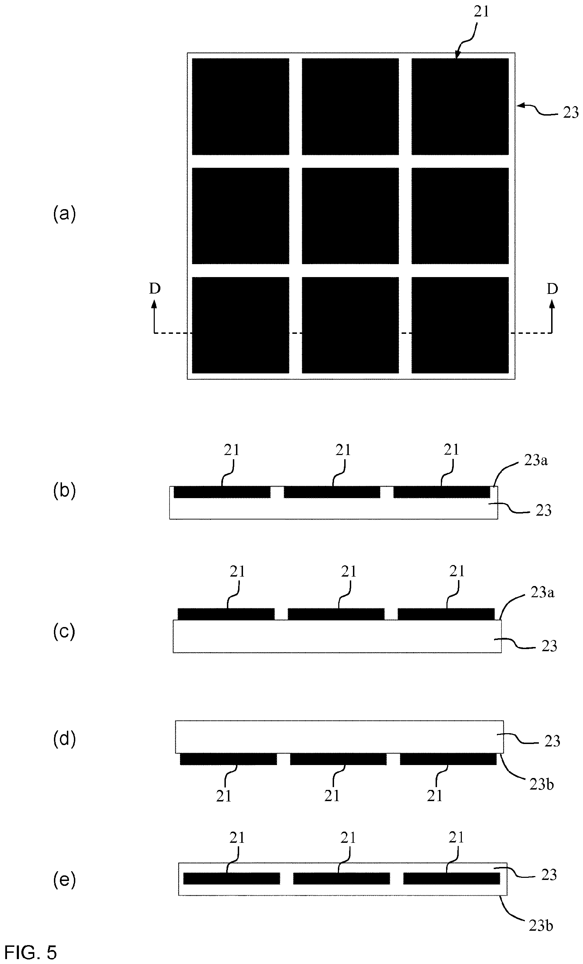

Next, a manufacturing method of a cell culture substrate will be described with reference to Figs. 5 to 8.

In one embodiment of the present invention, a manufacturing method of a cell culture substrate having a partition portion comprises: a non-adhesive portion transfer resin layer forming step, an adhesive portion resin layer forming step, and a composite resin layer forming step.

Each step will be described in more detail below.

### (1) Non-adhesive Portion Transfer Resin Layer Forming Step,

### (1-1) First Transferred Resin Layer Forming Step

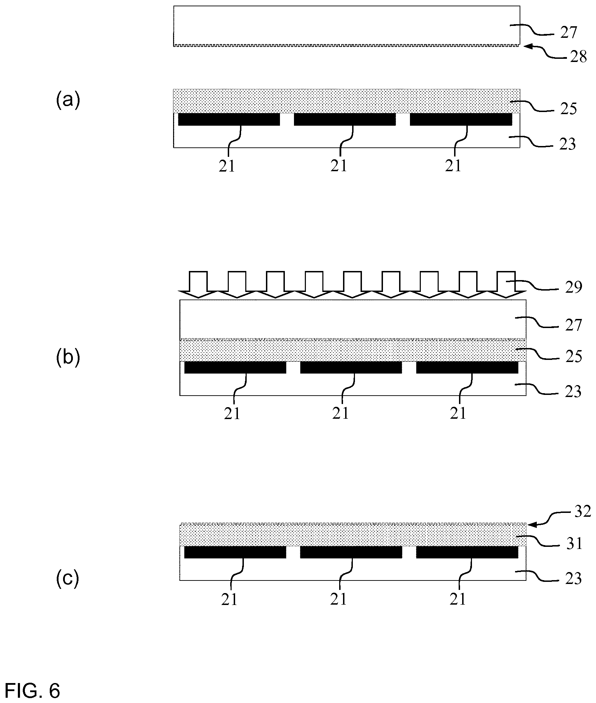

First, as illustrated in Fig. 6A, a first photocurable resin composition is applied on a transparent base 23 having a light shielding part 21 as shown in Fig. 5A to form a first transferred resin layer 25.

### <Transparent Base>

The transparent base 23 is formed from a transparent material, such as a resin base, a quartz base, and a silicone base. The material is preferably, but not particularly limited to, a resin base. This is because use of a resin base enables a cell culture substrate obtained in a desired size (available in a large area) by the method of the present invention. A resin constituting the resin base is made of, for example, one selected from the group consisting of polyethylene terephthalate, polycarbonate, polyester, polyolefin, polyimide, polysulfone, polyether sulfone, cyclic polyolefin, and polyethylene naphthalate. The transparent base 23 preferably has flexibility, and when such a resin base is used, may be a laminate of same or different bases or a laminate of a resin composition in a film form on the resin base. The resin base preferably has a thickness ranging from 25 to 500 µm.

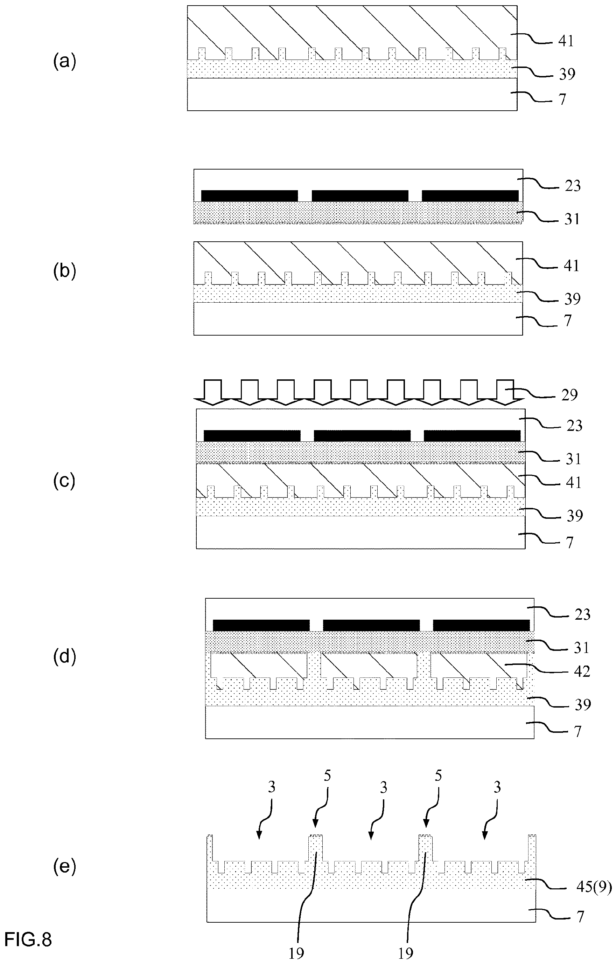

The light shielding part 21 provided in the transparent base 23 is a pattern utilized as a mask in the composite shape forming step. As illustrated in Figs. 8B to 8E, an adhesive portion 3 corresponding to the light shielding part 21, a partition portion 19, and a non-adhesive portion 5 on the partition portion are formed in a composite resin layer 45. In an active energy ray irradiation step illustrated in Fig. 8B, a region where active energy rays 29 is shielded by the light shielding part 21 becomes the adhesive portion 3. The "active energy ray" is the generic name for energy lines capable of curing a photocurable resin composition, such as UV light, visible light, and electron beams. The shape of the light shielding part 21 is not particularly limited, but may be a square shape as illustrated in Fig. 5A, a polygon such as a pentagon or a hexagon, a circle, or the like, and corresponds to the region of the adhesive portion 3.

The light shielding part 21 may be formed by patterning after deposition of a light shielding material (for example, a metal material, such as Cr) on the transparent base 23 by sputtering or formed by printing a pattern of a light shielding material by a method, such as ink jet printing and screen printing. As illustrated in Figs. 5B to 5C, the light shielding part 21 may be formed on the side of a surface 23a of the transparent base 23 to apply the first photocurable resin composition, or as illustrated in Fig. 5D, may be formed on a back side 23b of the transparent base 23. The light shielding part 21, as illustrated in Fig. 5B, may be formed flush with the transparent base 23, may be formed on a flat surface of the transparent base 23 as illustrated in Fig. 5C, or may be mounted in the transparent base 23 as illustrated in Fig. 5E.

### <First Photocurable Resin Composition>

The first photocurable resin composition constituting the first transferred resin layer 25 contains a monomer and a photoinitiator and is cured by irradiation with an active energy ray.

Examples of the monomer include photopolymerizable monomers to form a acrylic resin, a methacrylic resin, a styrene resin, an olefin resin, a polycarbonate resin, a polyester resin, an epoxy resin, a silicone resin, and the like, and a photopolymerizable acrylic monomer and/ or methacrylic monomer is preferred.

The photoinitiator is a component to be added to accelerate polymerization of a monomer and is preferably contained 0.1 parts by mass or more based on 100 parts by mass of the monomer. The upper limit of the photoinitiator content is not particularly defined but, for example, 20 parts by mass based on 100 parts by mass of the monomer.

The first photocurable resin composition of the present invention may contain components, such as a solvent, a polymerization inhibitor, a chain transfer agent, an antioxidant, a photosensitizer, a filler, and a leveling agent, without affecting the properties of the first photocurable resin composition.

The first photocurable resin composition may be manufactured by mixing the above components in a known method. The first photocurable resin composition may be applied on the transparent base 23 by a method, such as spin coating, spray coating, bar coating, dip coating, die coating, and slit coating, to form the first transferred resin layer 25.

The first transferred resin layer 25 is generally a transparent resin layer and has a thickness from 100 nm to 1 mm and preferably from 5 to 500 µm. With such a thickness, it is easy to perform imprinting processing and it is possible to form a reverse pattern of a non-adhesive portion.

### (1-2) Transfer and Curing Step

Then, as illustrated in Figs. 6A to 6C, with a first pattern 28 of a first mold 27 pressed against the first transferred resin layer 25, the first transferred resin layer 25 is irradiated with the active energy ray 29 through the first mold 27, and thereby the non-adhesive portion transfer resin layer 31 with the first pattern 28 transferred thereto is formed.

The first mold 27 has the first pattern 28. In the present embodiment, the first pattern 28 is a desired pattern of a non-adhesive portion 5.

The first mold 27 is made of a transparent material such as a resin base, a quartz base, and a silicone base, and can be formed of the same material as the transparent base 23.

The first mold 27 may be pressed against the first transferred resin layer 25 at a pressure that allows transfer of the shape of the first pattern 28 to the first transferred resin layer 25.

The first transferred resin layer 25 may be irradiated with the active energy ray 29 at an amount of the integral light to sufficiently cure the first transferred resin layer 25. The amount of the integral light is, for example, 100 to 10000 mJ/ cm². By the irradiation with the active energy ray 29, the first transferred resin layer 25 is cured to form, as illustrated in Fig. 6C, the first reverse pattern 32 in which the first pattern 28 is inverted, that is, the non-adhesive portion transfer resin layer 31 on which the inverted pattern of the desired non-adhesive portion 5 is formed.

### (2) Adhesive Portion Resin Layer Forming Step

### (2-1) Second Transferred Resin Layer Forming Step

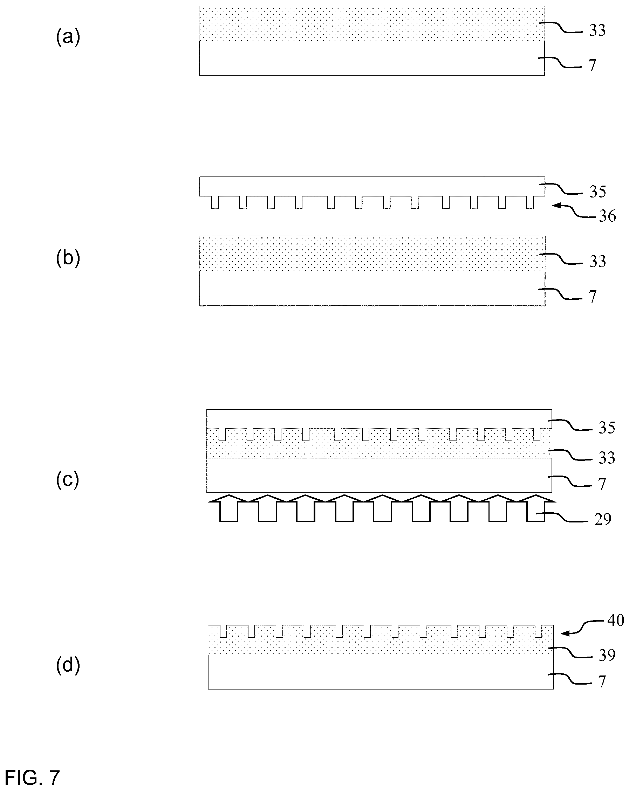

First, as illustrated in Fig. 7A, a second photocurable resin composition is applied on the base 7 to form a second transferred resin layer 33.

The above descriptions on the first photocurable resin composition apply to the second photocurable resin composition as long as not being inconsistent with the spirit. The type of the second photocurable resin composition may be same as or different from that of the first photocurable resin composition. The second transferred resin layer 33 obtained by applying the second photocurable resin composition is generally a transparent resin layer, and has a thickness of 1 µm to 1 mm, preferably 50 to 500 µm. With such a thickness, it is easy to perform imprinting processing and it is possible to form an adhesive portion.

### (2-2) Transfer and Curing Step

Then, as illustrated in Figs. 7B to 7D, with a second pattern 36 of a second mold 35 pressed against the second transferred resin layer 33, the second transferred resin layer 33 is irradiated with the active energy ray to cure the second transferred resin layer 33, and thereby the adhesive portion resin layer 39 is formed.

The second mold 35 has the second pattern 36. In the present embodiment, the second pattern 36 is a desired reverse pattern of an adhesive portion 3.

When the base 7 is transparent, irradiation with the active energy ray 29 may be performed from the side of the base. The second mold 35 may be formed of a transparent material such as a resin base, a quartz base, a silicone base, or a metal material.

The second mold 35 may be pressed against the second transferred resin layer 33 at a pressure that allows transfer of the shape of the second pattern 36 to the second transferred resin layer 33.

The second transferred resin layer 33 may be irradiated with the active energy ray 29 at an amount of the integral light to sufficiently cure the second transferred resin layer 33. The amount of the integral light is, for example, 100 to 10000 mJ/ cm². By the irradiation with the active energy ray 29, the second transferred resin layer 33 is cured to form, as illustrated in Fig. 7D, the second reverse pattern 40 in which the second pattern36 is inverted, that is, the adhesive portion resin layer 39 on which the desired non-adhesive portion 5 is formed.

### (3) Composite Resin Layer Forming Step

### (3-1) Third Transferred Resin Layer Forming Step

First, as illustrated in Fig. 8A, a third photocurable resin composition is applied on the adhesive portion resin layer 39 to form a third transferred resin layer 41.

The above descriptions on the first photocurable resin composition apply to the second photocurable resin composition as long as not being inconsistent with the spirit. The type of the third photocurable resin composition may be same as or different from that of the first photocurable resin composition, and may be same as or different from that of the second photocurable resin composition. the third transferred resin layer 41 obtained by applying the third photocurable resin composition is generally a transparent resin layer, and has a thickness of 10 µm to 1 mm, preferably 50 to 500 µm. With such a thickness, it is easy to perform imprinting processing and it is possible to form a partition portion and a non-adhesive portion.

### (3-2) Transfer and Curing Step

Then, as illustrated in Figs. 8B to 8E, with a non-adhesive portion tansfer resin layer 31 as a mold pressed against the third transferred resin layer 41, the third transferred resin layer 41 is irradiated with the active energy ray to cure the third transferred resin layer 41, and thereby the composite resin layer 45 is formed.

The non-adhesive portion tansfer resin layer 31 may be pressed against the third transferred resin layer 41 at a pressure that allows transfer of the shape of the non-adhesive portion tansfer resin layer 31 to the third transferred resin layer 41.

The third transferred resin layer 41 is irradiated with the active energy ray 29 the from the non-adhesive portion transfer resin layer 31.

The third transferred resin layer 41 may be irradiated with the active energy ray 29 at an amount of the integral light to sufficiently cure the third transferred resin layer 41. The amount of the integral light is, for example, 100 to 10000 mJ/ cm². By the irradiation with the active energy ray 29, in the region not shielded by the light shielding part 21, the third photocurable resin composition filled in the gap of the adhesive portion resin layer 39 is cured and the third transferred resin layer 41 to which the reverse pattern of the non-adhesive portion transfer resin layer 31 was transferred is cured, and thereby the composite resin layer 45 is formed. In this step, the partition portion 19 shown in Fig. 8E and the non-adhesive portion 5 on the partition portion 19 are formed in the region where the third photocurable resin composition was cured. On the other hand, the adhesive portion 3 remains intact in the region where the active energy ray 29 was shielded by the light shielding part 21 and the third photocurable resin composition was not cured.

Then, as illustrated in Figs. 8D to 8E, the non-adhesive portion tansfer resin layer 31 is removed and third photocurable resin composition 42 remaining on the adhesive portion 3 is removed with solvent to obtain the structure shown in Fig. 8E, and the production of the cell culture substrate is completed.

By using the composite resin layer 45 formed above, it is possible to prepare a mold having the reverse pattern of the composite resin layer 45, and by using this mold, a cell culture substrate having the same pattern as that of the composite resin layer 45 can be parpared at once.

In the case of not having the partition portion portion, it is possible to manufacture the cell culture substrate as shown in Fig. 4B by using a mold having the adhesive portion 3 and the reverse pattern of the non-adhesive portion 5.

### EXAMPLES

### 1. Preparation of Cell Culture Substrate Sample

Hereinafter, the preparation of the cell culture substrate sample used for cell culture and the evaluation of drug metabolism will be described.

### < Preparation of Photocurable Resin Composition >

First, a photocurable resin composition was prepared by blending a photopolymerizable monomer and a photoinitiator in the proportions shown below.

### Photopolymerizable Monomer:

Ethylene oxide modified trimethylolpropane triacrylate (Manufactured by Osaka Organic Chemical Industry Ltd.; Name of product: Viscoat # 360): 50 parts by mass
Ethylene oxide modified bisphenol A diacrylate (Manufactured by Osaka Organic Chemical Industry Ltd.; Name of product: Viscoat # 700HV): 20 parts by mass
Tripropylene glycol diacrylate (Manufactured by Osaka Organic Chemical Industry Ltd.; Name of product: Viscoat # 310HP): 30 parts by mass

### Photoinitiator:

1-hydroxycyclohexyl phenyl ketone (Manufactured by BASF Japan; product name: Irgacure 184): 5 parts by mass

### < Preparation of Sample >

### [Sample 1] (Example 1)

### (Formation of Non-adhesive Portion Transfer Resin Layer)

As illustrated in Fig. 5, the photocurable resin composition prepared above was applied to the PET base 47 provided with the light shielding part 21 as a plurality of squares at a thickness of 10 µm with a bar coater, and a lamination was carried out on a mold of nanopillar having a circular column shape (pitch: 150 nm, height: 250 nm, diameter: 100 nm) with a roller from above in such a manner that the coated resin surface is pressed against the mold. Thereafter, UV irradiation was performed from the mold side at an amount of the integral light of 500 mJ/ cm² to cure the photocurable resin composition. The mold and the resin-cured PET base were peeled off to prepare a nanohole transfer resin layer having the reverse shape of the mold.

### (Formation of Adhesive Portion Resin Layer)

The photocurable resin composition prepared above was applied to the PET base at a thickness of 10 µm with a bar coater, and a lamination was carried out on a mold of nanohole having a hexagonal columnar shape (honeycomb) (pitch :12 µm, depth: 5 µm, parallel two side width: 17 µm) with a roller from above in such a manner that the coated resin surface is pressed against the mold. Thereafter, UV irradiation was performed from the PET base side at an amount of the integral light of 500mJ / cm² to cure the photocurable resin composition. The mold and the resin-cured PET base were peeled off to prepare a nanopillar transfer resin layer having the reverse shape of the mold.

### (Formation of Composite Resin Layer)

The photocurable resin composition prepared above was applied to the nanopillar transfer resin layer at a thickness of 10 µm with a bar coater, and a lamination was carried out on the nanohole transfer resin layer from above using the nanohole transfer resin layer as a mold in such a manner that the coated resin surface is pressed against the nanohole transfer resin layer. Thereafter, UV irradiation was performed from the nanohole transfer resin layer at an amount of the integral light of 500 mJ/ cm² to cure the photocurable resin composition. The nanohole transfer resin layer and the nanopillar transfer resin layer were peeled off, and the uncured photocurable resin composition remaining on the nanopillar transfer resin layer was removed with isopropyl alcohol to prepare a cell culture substrate.

### [Sample 2] (Comparative Example 1)

A cell culture substrate was prepared in the same manner as in the composite resin layer forming step of Sample 1, except that instead of using the nanohole transfer resin layer as the mold, a flat one having the same light shielding part 21 in lattice shape and not having the concave-convex shape was used.

### [Sample 3] (Comparative Example 2)

A cell culture substrate was prepared only by the adhesive portion resin layer forming step of Sample 1.

### [Sample 4] (Comparative Example 3)

The photocurable resin composition prepared above was applied to the PET base at a thickness of 10 µm with a bar coater, and UV irradiation was performed from the PET base side at an amount of the integral light of 500 mJ/ cm² to cure the photocurable resin composition.

### <Measurement of Each Part of Sample 1 >

A part of Sample 1 was excised, the shape was observed using a scanning electron microscope (model: JSM-7800F, manufactured by JEOL Ltd.), and each part was measured using software (PC-SUM) attached to the same microscope. The results for the adhesive portion and the non-adhesive portion are shown in Table 1, and the results for the partition portion are shown in Table 2. In the sample 1, the upper surface area of the partition portion is substantially equal to the area of the region of the non-adhesive portion.

**[Table 1]**

| Table 1 | Adhesive Portion | Non-adhesive Portion |
|---|---|---|
| Shape | Hexagonal Column | Circular Column |
| Pitch | 12 µm | 150 nm |
| Height | 5 µm | 250 nm |
| Width / Diameter | 10 µm | 100 nm |
| Upper Surface Area | 195 µm² | 10000πnm² |

**[Table 2]**

| Table 1 | Partition Portion |
|---|---|
| Width | 3 µm |
| Height | 12 µm |
| Per Unit Area of Separated Adhesion Portion | 95×95 µm² |
| Per Unit Area of Upper Surface of Partition Portion | 2000 µm² |

### < Measurement of Contact Angle of Water >

With respect to the obtained Sample 1, 0.5µl of ion-exchanged water was dropped on the surface of the adhesive portion and the non-adhesive portion of Sample 1, and the contact angle of water (water contact angle) was measured at 25°C using a contact angle measuring device (manufactured by dataphysics) and found to be 100° and 115° respectively.

### 2. Cell Culture and Drug Metabolism

The results of the cell culture and drug metabolism using the cell culture substrate of the present invention will be described below.

### < Cell Culture Method >

The cell culture substrate was placed on the bottom of a 6 -well plate (manufactured by Thermo Fisher Scientific) and immersed in 70% ethanol for 1 hour. Then, it was washed three times with PBS having a pH of 7.4 (hereinafter the same) and dried.

Next, rat hepatocytes were suspended in DMEM medium (manufactured by Thermo Fisher Scientific), seeded in a well plate at 1 × 10⁵ cells / well and cultured at 37°C under 5% CO₂ for 14 days. The medium was exchanged the next day of culture, and thereafter it was exchanged every other day.

### < Aggregated Shape >

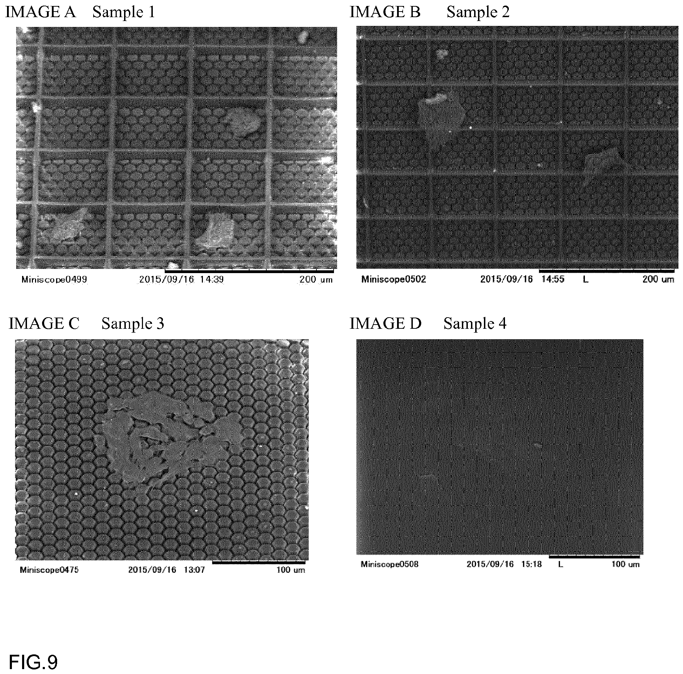

In order to investigate the formation of spheroids, the aggregated shape after 14 days after seeding was observed using SEM. SEM images in the case of using Samples 1 to 4 are shown in Figs. 9A to 9D.

In Sample 1, the cells aggregated in a spherical shape in the lattice, the size of each aggregate was close, and uniform spheroids were formed. In Sample 2, the cells aggregated to some extent but not in a spherical shape as they were crossing the lattice, and it can not be said that their sizes were uniform. Unlike Sample 1, it is presumed that it may be caused by not having a non-adhesive portion on the lattice. In Sample 3, although the cells aggregated, they were only somewhat rounded and their sizes were largely different from each other. It is presumed that this is because the adhesive portion was not surrounded by a non-adhesive portion and there was no partition portion. In sample 4, the cells only spread thinly.

### < Evaluation of Drug Metabolism >

The evaluation of drug metabolism was performed 1, 7 and 14 days after seeding. After 1, 7 and 14 days, replace medium with induction medium (3-MC 0.333 mM) and incubate for 24 hours. Subsequently, the medium was replaced with a reaction medium (PBS 50 ml, Dicumarol 25 µM, Probenecid 2 mM, ethoxyresorufin 20 µM) heated to 37 ° C, after 60 minutes of incubation, the pH was adjusted to 7.4 with HCl. 200 µl of supernatant and 200 µl of blank (unreacted reaction medium) were placed in a 96-well plate, and the amount of fluorescence [530/590] was measured with a fluorescence plate reader.

The evaluation of drug metabolism of Samples 1 to 4 is shown in Table 3.

**[Table 3]**

| Table 3 | Samples | Amount of Drug Metabolism (µM/1×10⁵ cells) | | |
|---|---|---|---|---|
| | | 1 day later | 7 days later | 14 days later |
| Example 1 | 1 | 4.8 | 6.4 | 7.8 |
| Comparative Example 1 | 2 | 2.5 | 3.2 | 6.6 |
| Comparative Example 2 | 3 | 3.7 | 4.0 | 5.2 |
| Comparative Example 3 | 4 | 2.7 | 2.5 | 2.6 |

In sample 1, it is estimated that the amount of drug metabolism is large, and the oxygen supply is sufficiently carried out on the spheroid being formed. In Sample 2, although the amount of drug metabolism was relatively large, it was inferior to Sample 1. In Sample 3, the amount of drug metabolism was as little as 30 to 40% of Sample 1. In Sample 4, the amount of drug metabolism was greatly reduced.

### Reference Signs List

1: Cell Culture Substrate, 3: Adhesive Portion, 5: Non-Adhesive Portion, 19: Partition portion, 29: Active Energy Ray, P₁: Pitch of Concave-Convex Shape of Adhesive Portion, P₂: Pitch of Concave-Convex Shape of Non-Adhesive Portion, G₂: Gap Between Two Adjacent Columnar Convex Portions

## Claims

1. A cell culture substrate having an adhesive portion and a non-adhesive portion, wherein
the adhesive portion and the non-adhesive portion have concave-convex shapes, and
a contact angle of pure water on the non-adhesive portion is larger than that of the adhesive portion.

2. The cell culture substrate of Claim 1, wherein
pitch P₁ of the concave-convex shape of the adhesive portion is larger than pitch P₂ of the concave-convex shape of the non-adhesive portion.

3. The cell culture substrate of Claim 2, wherein
the ratio P₁/P₂ of the pitch P₁ and the pitch P₂ is 2 or more.

4. The cell culture substrate of any one of Claims 1 to 3, wherein
at least a part of the adhesive portion is separated by a partition portion, and
the non-adhesive portion is formed on the partition portion.

5. The cell culture substrate of any one of Claims 2 to 4, wherein
the non-adhesive portion has a plurality of columnar convex portions,
the pitch at which the columnar convex portion of the non-adhesive portion is formed is 50 to 2000 nm,
the columnar convex portion of the non-adhesive portion has an upper surface area of 1600 nm² to 4 µm², and
the gap G₂ between two adjacent columnar convex portions is in the range of 10 to 1960 nm.

6. The cell culture substrate of any one of Claims 1 to 5, wherein
the adhesive portion and the non-adhesive portion are made of a same material.

7. A manufacturing method of the cell culture substrate of Claims 1 to 6, comprising
a step of irradiating, with an active energy ray, a photocurable resin composition coated on a substrate to cure the resin composition to form concave-convex shapes of the adhesive portion and the non-adhesive portion.

8. The manufacturing method of the cell culture substrate of Claim 7, wherein
the concave-convex shapes of the adhesive portion and the non-adhesive portion are formed by a nanoimprint method using a mold.
